# EUROPEAN PATENT APPLICATION

(11) **EP 3 273 228 A1**
(43) Date of publication of application: **24.01.2018**
(21) Application number: 17182305.7
(22) Date of filing: 20.07.2017
(51) Int. Cl.: G01N 21/67, G01N 21/69, G01N 33/20, G01N 1/40

(54) **PLASMA SPECTROSCOPIC ANALYSIS METHOD AND PLASMA SPECTROSCOPIC ANALYZER**

(30) Priority: 22.07.2016 JP 2016144930; 23.06.2017 JP 2017123020
(71) Applicant: ARKRAY, Inc., Minami-ku Kyoto-shi, Kyoto 601-8045 (JP)
(72) Inventor: TAKASU, Tsuyoshi, Kyoto, 602-0008 (JP)
(74) Representative: Hoffmann Eitle

(57) **Abstract**

The plasma spectroscopic analysis method includes: a concentration process including a voltage application period in which voltage is applied to a pair of electrodes in the presence of a sample thereby concentrating an analyte, contained in the sample, in the vicinity of at least one of the pair of electrodes; and
a detection process of generating a plasma by applying voltage to the pair of electrodes and detecting light emitted by the analyte due to the plasma,
wherein an electric current between the pair of electrodes is constant while applying voltage for at least a part of the duration of the concentration process.

## Description

### BACKGROUND

### Technical Field

The present disclosure relates to a plasma spectroscopic analysis method and a plasma spectroscopic analyzer.

### Background Art

As methods of analyzing an analyte contained in a sample, analysis methods utilizing plasma emission are known.

Regarding the analysis methods, Japanese Patent Application Laid-Open (JP-A) No. 2009-128315 discloses a method of analyzing a sample using a radiofrequency plasma mass spectrometer. Further, JP-A No. 2011-180045 and JP-A No. 2012-185064 disclose a method of analyzing a sample by generating a plasma in the sample using a plasma generator including a narrow section and analyzing the plasma emission. Moreover, WO 2006/059808 and WO 2011/099247 disclose a method of analyzing a sample by generating a plasma in a liquid sample and analyzing the plasma emission.

However, in the method of JP-A No. 2009-128315, there was a problem that, without a proper pretreatment, contamination of the sample with other substances caused variations in the analysis results. Further, in the methods of JP-A No. 2011-180045 and JP-A No. 2012-185064, when a sample containing impurities is used or when foreign substances and the like are mixed into a liquid sample during a pretreatment of reducing the amount of the liquid sample, there was a problem that the narrow section is clogged with the impurities or the foreign substances, making it difficult to perform the measurement. The methods of WO 2006/059808 and WO 2011/099247 also had a problem that the analytical sensitivity was low.

### SUMMARY OF INVENTION

### Technical Problem

In view of the above, an object of the present disclosure is to provide: a plasma spectroscopic analysis method having a high analytical sensitivity and by which, for example, occurrences of analytical errors in sample analysis is suppressed; and a plasma spectroscopic analyzer for carrying out the plasma spectroscopic analysis method.

### Solution to Problem

In order to solve the above-described problems, the plasma spectroscopic analysis method according to the present disclosure (hereinafter, also referred to as "analysis method") is characterized by including:
a concentration process including a voltage application period in which voltage is applied to a pair of electrodes in the presence of a sample, thereby concentrating an analyte, contained in the sample, in the vicinity of at least one of the pair of electrodes; and
a detection process of generating a plasma by applying voltage to the pair of electrodes and detecting light emitted by the analyte due to the plasma,
wherein an electric current between the pair of electrodes is constant while applying voltage for at least a part of the duration of the concentration process.

The plasma spectroscopic analyzer according to the present disclosure (hereinafter, also referred to as "analyzer") is characterized by including:
a pair of electrodes;
a container;
a light-receiving section; and
a constant current section, wherein,
the container includes a light-transmitting section,
the pair of electrodes is placed inside the container,
the light-receiving section, which is capable of receiving, via the light-transmitting section, light emitted by an analyte due to voltage application to the pair of electrodes, is placed outside the container,
the constant current section is connected to the pair of electrodes and controls an electric current between the pair of electrodes so as to be constant during the voltage application to the pair of electrodes, and
the plasma spectroscopic analyzer is used for carrying out the plasma spectroscopic analysis method according to the present disclosure.

### Effects of the Invention

According to the plasma spectroscopic analysis method in the present disclosure, a high analytical sensitivity can be achieved and, for example, occurrences of analytical errors in sample analysis can be suppressed.

### BRIEF DESCRIPTION OF DRAWINGS

FIG. 1A is a schematic perspective view which illustrates one embodiment of the analyzer according to the present disclosure.
FIG. 1B is a schematic cross-sectional view taken in the direction of I-I as shown in FIG. 1A.
FIG. 2 is a graph showing a correlation between the lead concentration and the number of counts in Example 1.
FIG. 3 is a graph showing the number of counts obtained in Example 2 at respective cycles.

### DESCRIPTION OF EMBODIMENTS

### <Plasma Spectroscopic Analysis Method>

As described above, the plasma spectroscopic analysis method according to the present disclosure includes: a concentration process including a voltage application period in which voltage is applied to a pair of electrodes in the presence of a sample, thereby concentrating an analyte, contained in the sample, in the vicinity of at least one of the pair of electrodes; and
a detection process of generating a plasma by applying voltage to the pair of electrodes and detecting light emitted by the analyte due to the plasma,
wherein an electric current between the pair of electrodes is constant while applying voltage for at least a part of the duration of the concentration process. The analysis method according to the present disclosure is characterized in that it includes the concentration process and the detection process and that an electric current between the pair of electrodes is constant while applying voltage for at least a part of the duration of the concentration process, and other processes and conditions are not particularly restricted.

Generally, in order to efficiently analyze an analyte contained in a sample, for example, the amount of the analyte per unit volume of the whole sample is increased by subjecting the sample to a pretreatment of reducing the total volume (total liquid volume) thereof by concentration. In contrast, the inventors discovered that, by applying voltage to a pair of electrodes in the presence of a sample so as to concentrate an analyte, contained in the sample, in the vicinity of at least one of the pair of electrodes, that is, to accumulate the analyte locally in the vicinity of the electrode, and further generating a plasma at the electrode around which the analyte has thus been accumulated, the analyte locally accumulated at a high concentration can be efficiently analyzed, thereby establishing a novel analysis method which does not require a pretreatment described above. However, the use of this analysis method sometimes yields varying analysis results among plural samples containing the same concentration of analyte. The inventors intensively studied to discover that, for example, in samples which contain an analyte (e.g., lead) and a certain co-existing substance such as ethylenediamine tetraacetic acid (EDTA: chelating agent), errors occur in analysis results, which leads to varying analysis results among plural samples. In other words, the inventors discovered that the varying analysis results among plural samples are attributed to occurrences of analysis errors caused by the presence of a certain co-existing substance. Moreover, the inventors also discovered that, although the mechanism is unclear, even when the samples contain a certain co-existing substance such as the substance described above, the occurrences of analytical errors can be suppressed by controlling the electric current between the pair of electrodes so as to be constant while applying voltage for at least a part of the duration of the concentration process, that is, employing a constant electric current, and completed the analysis method according to the present disclosure. Therefore, according to the analysis method in the present disclosure, for example, occurrences of analytical errors in sample analysis can be suppressed. Further, in the analysis method according to the present disclosure, since occurrences of analytical errors in sample analysis can be suppressed, an analyte can be accurately analyzed regardless of, for example, the type of the sample and the type of the analyte. Moreover, as described above, since the analysis method according to the present disclosure can efficiently analyze an analyte locally accumulated at a high concentration without performing the above-described pretreatment on the sample, the sample can be analyzed by a simple method with high sensitivity.

In the analysis method according to the present disclosure, the sample is, for example, a specimen. The specimen may be a liquid specimen or a solid specimen. For example, an undiluted liquid specimen may be directly used as a liquid specimen, or a diluted liquid obtained by suspending, dispersing or dissolving the specimen in a medium may be used as a liquid specimen. When the specimen is a solid, for example, it is preferable to use a diluted liquid obtained by suspending, dispersing or dissolving the specimen in a medium as a liquid specimen. The medium is not particularly restricted, and examples thereof include water and a buffer. The specimen is, for example, a specimen (sample) derived from a biological source, a specimen (sample) derived from an environmental source, a metal, a chemical substance, or a pharmaceutical agent. Examples of the specimen derived from a biological source include, but not particularly limited to, specimens from urine, blood, hair, saliva, sweat, and a nail. Examples of the blood specimen include erythrocytes, whole blood, serum, and blood plasma. Examples of the biological source include humans, non-human animals, and plants, and examples of the non-human animals include mammals excluding humans, fish, and shellfish. Examples of the specimen derived from an environmental source include, but not particularly limited to, foodstuff, water, soil, the atmosphere, and air. Examples of the foodstuff include fresh food and processed food. Examples of the water include drinking water, underground water, river water, sea water, and domestic wastewater. The sample contains, for example, a metal and a chelating agent described below, and preferably the metal is the analyte.

The analyte is not particularly restricted and may be, for example, a metal or a chemical substance. Examples of the metal include, but not particularly limited to, aluminum (Al), antimony (Sb), arsenic (As), barium (Ba), beryllium (Be), bismuth (Bi), cadmium (Cd), cesium (Cs), gadolinium (Gd), lead (Pb), mercury (Hg), nickel (Ni), palladium (Pd), platinum (Pt), tellurium (Te), thallium (Tl), thorium (Th), tin (Sn), tungsten (W), and uranium (U). Examples of the chemical substance include a reagent, an agricultural chemical, and cosmetics. The analyte may, for example, consist of one substance, and may include two or more substances.

When the analyte is a metal, the sample may contain, for example, a reagent for separating the metal contained in the specimen. The reagent may be, for example, a chelating agent or a masking agent. Examples of the chelating agent include dithizone, tiopronin, meso-2,3-dimercaptosuccinic acid (DMSA), sodium 2,3-dimercapto-1-propanesulfonate (DMPS), ethylenediamine tetraacetic acid (EDTA), nitrilotriacetic acid (NTA), ethylenediamine-*N,N*'-disuccinic acid (EDDS), and *α*-lipoic acid. In the present disclosure, "masking" means inactivation of the reactivity of an SH group, which can be performed by, for example, chemical modification of the SH group. Examples of the masking agent include maleimide, *N*-methylmaleimide, *N*-ethylmaleimide, *N*-phenylmaleimide, maleimidopropionic acid, iodoacetamide, and iodoacetic acid.

The sample may also be, for example, a sample whose pH has been adjusted (hereinafter, also referred to as "pH-adjusted sample"). The pH of the pH-adjusted sample is not particularly restricted. The method of adjusting the pH of the sample is not particularly restricted and, for example, a pH-adjusting reagent such as an alkaline reagent or an acidic reagent may be used.

The alkaline reagent may be, for example, an alkali or an aqueous solution thereof. Examples of the alkali include, but not particularly limited to, sodium hydroxide, lithium hydroxide, potassium hydroxide, and ammonia. Examples of the aqueous solution of the alkali include solutions obtained by diluting the alkali with water or a buffer. In the aqueous solution of the alkali, the alkali concentration is not particularly restricted and may be, for example, from 0.01 mol/L to 5 mol/L.

The acidic reagent may be, for example, an acid or an aqueous solution thereof. Examples of the acid include, but not particularly limited to, hydrochloric acid, sulfuric acid, acetic acid, boric acid, phosphoric acid, citric acid, malic acid, succinic acid, and nitric acid. Examples of the aqueous solution of the acid include solutions obtained by diluting the acid with water or a buffer. In the aqueous solution of the acid, the acid concentration is not particularly restricted and may be, for example, from 0.01 mol/L to 5 mol/L.

The electrodes are not particularly restricted and may be, for example, solid electrodes, and specific examples thereof include rod electrodes and spherical electrodes. The material of the electrodes is also not particularly restricted as long as it is a solid electroconductive material, and the material of the electrodes can be determined as appropriate in accordance with, for example, types of the analyte. The material of the electrodes may be, for example, a nonmetal, a metal, or a mixture thereof. When the material of the electrodes is a nonmetal-containing material, for example, the material of the electrodes may contain a single type of nonmetal, and may contain two or more types of nonmetals. Examples of the nonmetal include carbon. When the material of the electrodes is a metal-containing material, for example, the material of the electrodes may contain a single type of metal, and may contain two or more types of metals. Examples of the metal include gold, platinum, copper, zinc, tin, nickel, palladium, titanium, molybdenum, chromium, and iron. When the material of the electrodes contains two or more types of metals, the material of the electrodes may be an alloy. Examples of the alloy include brass, steel, INCONEL^{™}, nichrome, and stainless steel. The material for the pair of electrodes may be, for example, the same or may be different.

The size of respective electrodes is not particularly restricted as long as it is, for example, a size which allows the electrodes to be in contact with the sample. When an electrode is a rod electrode, the diameter of the electrode is, for example, preferably from 0.02 mm to 50 mm, more preferably from 0.05 mm to 5 mm. The length of each electrode is, for example, preferably from 0.1 mm to 200 mm, more preferably from 0.3 mm to 50 mm.

As described above, the concentration process is a process including a voltage application period in which voltage is applied to a pair of electrodes in the presence of a sample, thereby concentrating an analyte, contained in the sample, in the vicinity of at least one of the pair of electrodes, wherein an electric current between the pair of electrodes is constant while applying voltage for at least a part of the duration of the concentration process. For example, the pair of electrodes is in contact (wetted) with the sample. In the concentration process, the expression "concentrating an analyte, contained in the sample, in the vicinity of at least one of the pair of electrodes" means to electrically attracting the analyte contained in the sample to the vicinity of an electrode. The range of the "vicinity of an electrode" is not particularly restricted and may be, for example, a range where a plasma is generated in the below-described detection process. In the present disclosure, the term "vicinity of an electrode" also encompasses parts on the electrode (i.e., parts that are in contact with the electrode).

In the concentration process, the expression "an electric current between the pair of electrodes is constant" means that the electric current between the electrodes is a constant electric current. In the present disclosure, the expressions "an electric current is constant" and "a constant electric current" encompass cases in which the electric current between the electrodes is substantially constant. The cases in which the electric current between the electrodes is substantially constant refer to cases in which, even if the electric current value fluctuates over time from a preset electric current value, the electric current value between the electrodes (Ac) is maintained within the range of ± 20% of the preset electric current value (As) (i.e., 0.8 × As ≤ Ac ≤ 1.2× As). Examples of the cases in which "an electric current is constant" or in which the electric current is "a constant electric current" include cases in which the electric current between the electrodes (Ac) is maintained within the range of ± 10% of the preset electric current value (As) (i.e., 0.9 × As ≤ Ac ≤ 1.1 × As) and in which the electric current between the electrodes (Ac) is maintained within the range of ± 5% of the preset electric current value (As) (i.e., 0.95 × As ≤ Ac ≤ 1.05 × As). The description regarding the electric current between the pair of electrodes described below, for example, may be applied to the preset electric current value.

In the analysis method according to the present disclosure, when the electric current between the pair of electrodes is constant while applying voltage for at least a part of the duration of the concentration process, occurrences of analytical errors in the sample analysis can be suppressed. For example, when analyzing a sample containing a co-existing substance (e.g., EDTA) and a sample not containing the co-existing substance, both containing the same concentration of an analyte (e.g., Pb), using an analysis method according to the present disclosure, an analytical error between the measured value of the concentration of the analyte in the sample containing the co-existing substance and that in the sample not containing the co-existing substance can be suppressed. For example, the error can be suppressed within the range of ± 15%, preferably ± 10%, and more preferably ± 15%, of a reference value. The reference value may be determined as appropriate using known methods.

In the concentration process, for example, the analyte may be partially concentrated in the vicinity of the electrode, and may be entirely concentrated in the vicinity of the electrode.

In the concentration process, it is preferable to set the electric charge conditions of the electrodes such that, in the below-described detection process, the analyte is concentrated on the electrode used for the detection of the analyte (i.e., the electrode used for plasma generation). Electric charge conditions are not particularly restricted and, for example, when the analyte is positively charged, the electric charge conditions may be set such that the electrode used for plasma generation is negatively charged. Further, for example, when the analyte is negatively charged, the electric charge conditions may be set such that the electrode used for plasma generation is positively charged.

The concentration process of the analyte can be controlled by, for example, adjusting the voltage. Thus, those of ordinary skill in the art would be able to appropriately set the voltage at which the concentration takes place (hereinafter, also referred to as "concentration voltage"). The concentration voltage may be, for example, 1 mV or higher, or 400 mV or higher. The upper limit of the concentration voltage is not particularly restricted and may be, for example, 1000V or less. The concentration voltage may be, for example, constant throughout the period of the concentration process, and may be changed during the concentration process. The concentration voltage may also be, for example, voltage at which no plasma is generated.

The duration of applying the concentration voltage (hereinafter, also referred to as "application time" in the concentration process) is not particularly restricted and may be set as appropriate in accordance with the concentration voltage. The duration of applying the concentration voltage is, for example, preferably from 0.2 to 40 minutes, more preferably from 1 to 5 minutes. The voltage application to the pair of electrodes may be performed, for example, continuously or discontinuously. The discontinuous voltage application is, for example, pulse application. When the voltage application is performed discontinuously, the duration of applying the concentration voltage may either be defined as a total time of applying the concentration voltage, or as a total of the time of applying the concentration voltage and the time of not applying the concentration voltage.

In the analysis method according to the present disclosure, an electric current between the pair of electrodes is constant while applying voltage for at least a part of the duration of the concentration process. The duration during which the electric current between the pair of electrode is constant is preferably 50% or longer, more preferably 70% or longer, still more preferably 80% or longer, and particularly preferably 90% or longer, and extremely preferably 100%, with respect to the total duration of the time of applying a concentration voltage.

The voltage application to the electrodes can be performed using a voltage application means. The voltage application means is not particularly restricted as long as it is capable of applying voltage such that the electric current between the pair of electrodes is controlled to be a constant electric current, and any known means such as a voltage generator can be used.

In the concentration process, the electric current between the pair of electrodes is, for example, preferably from 0.01 mA to 200 mA, more preferably from 10 mA to 60 mA, still more preferably from 10 mA to 40 mA. The electric current may be particularly preferably set to be 10 mA, 20 mA, or 40 mA.

In the concentration process, the voltage application to the pair of electrodes is preferably performed discontinuously since, for example, the above-described occurrences of analysis errors can be efficiently suppressed. When the voltage application to the pair of electrodes is performed discontinuously, the concentration process includes, for example: a voltage application period in which voltage is applied to the pair of electrodes; and a voltage non-application period in which no voltage is applied to the pair of electrodes. In this case, the electric current between the pair of electrodes is constant during at least a part of at least one voltage application period.

In the voltage application period, by applying voltage to the pair of electrodes, for example, the analyte contained in the sample is concentrated in the vicinity of at least one of the pair of electrodes. Therefore, in the voltage application period, it is preferable to set the electric charge conditions of the electrodes such that, in the below-described detection process, the analyte is concentrated on the electrode used for the detection of the analyte (i.e., the electrode used for plasma generation). Regarding the voltage applied to the pair of electrodes in the voltage application period, for example, the above-provided descriptions on the concentration voltage can be applied. In the voltage application period, the electric current between the pair of electrodes is, for example, preferably from 0.01 mA to 200 mA, more preferably from 10 mA to 60 mA, still more preferably from 10 mA to 40 mA. The electric current may be particularly preferably set to be 10 mA, 20 mA, or 40 mA.

The concentration process may include, for example, one voltage application period, and may include more than one voltage application periods. When the concentration process includes more than one voltage application period, the electric current between the pair of electrodes is constant during at least a part of at least one voltage application period. It is preferable that the electric current between the pair of electrodes is constant throughout at least one voltage application period. The electric current value between the pair of electrode at respective voltage application periods may be the same and may be different, and preferably the same. It is more preferable that, while applying voltage, the electric current between the pair of electrodes is constant (i.e., is a constant electric current) and the electric current values are the same through more than one voltage application periods.

In the voltage non-application period, no voltage is applied to the pair of electrodes. Therefore, in the voltage non-application period, for example, concentration of the analyte to the vicinity of at least one of the pair of electrodes does not take place. In the voltage non-application period, the voltage applied to the pair of electrodes is, for example, 0 V. Further, in the voltage application period, the electric current between the pair of electrodes may be set at 0 mA. These exemplified values of the voltage and the electric current that are applied to the pair of electrodes in the voltage non-application period are, for example, values of the voltage and the electric current that are applied to the electrodes from outside of the electrodes. Accordingly, there may be an electric potential difference between the pair of electrodes depending on, for example, the material of the electrodes, types of the sample, or conditions of the sample.

The concentration process may include, for example, one voltage non-application period, and may include more than one voltage non-application periods. The number of voltage non-application periods included in the concentration process may be, for example, the same as that of the voltage application period, and may be different from that of the voltage application period. It is preferable that the number of voltage non-application period included in the concentration process is the same as that of the voltage application period.

Voltage application in the voltage application period and voltage non-application in the voltage non-application period can be performed by, for example, adjusting the voltage to be applied. For the adjustment of the voltage to be applied, for example, a method of switching the electric circuit between a closed circuit and an open circuit can be employed.

In cases where the electric circuit is switched between a closed circuit and an open circuit, for example, the voltage application period and the voltage non-application period are alternately switched by switching the electric circuit between a closed circuit and an open circuit. The closed-circuit state corresponds to the voltage application period, and voltage can be applied to the pair of electrodes by allowing the electric circuit to be a closed circuit. Meanwhile, the open-circuit state corresponds to the voltage non-application period and, by switching the electric circuit to be an open circuit, the voltage application can be unapplied, that is, the voltage can be adjusted to be 0 V. The voltage of the closed circuit is the voltage in the voltage application period (i.e., the concentration voltage), and the voltage of the open circuit (i.e., 0 V) is the voltage in the voltage non-application period, meaning that no voltage is applied to the pair of electrodes in the voltage non-application period. The voltage of the closed circuit is not particularly restricted and, for example, the values exemplified above for the concentration voltage can be applied.

In the concentration process, when a cycle of one voltage application period and one voltage non-application period is defined as a single set, the duration of the single set is not particularly restricted. The duration of the single set is hereinafter also referred to as "application cycle". The lower limit of the application cycle is, for example, preferably 250 ms or longer, more preferably 1,000 ms or longer and, from the viewpoint of further improving the analytical sensitivity, the lower limit of the application cycle is still more preferably 2,000 ms or longer, particularly preferably 3,000 ms or longer. Further, the upper limit of the application cycle is, for example, preferably 600,000 ms or shorter, more preferably 64,000 ms or shorter, still more preferably 10,000 ms or shorter. The application cycle is in a range of, for example, preferably from 250 ms to 600,000 ms, more preferably from 1,000 ms to 600,000 ms, still more preferably from 2,000 ms to 600,000 ms, particularly preferably from 2,000 ms to 64,000 ms, extremely preferably from 2,000 ms to 10,000 ms.

In the concentration process, when a cycle of one voltage application period and one voltage non-application period is defined as a single set, the duration of the voltage non-application period in the single set is not particularly restricted. The duration of the voltage non-application period is hereinafter also referred to as "non-application time". The lower limit of the non-application time is, for example, preferably 125 ms or longer, more preferably 1,000 ms or longer, still more preferably 1,500 ms or longer. Further, the upper limit of the non-application time is, for example, preferably 300,000 ms or shorter, more preferably 32,000 ms or shorter, still more preferably 10,000 ms or shorter. The non-application time is in a range of, for example, preferably from 125 ms to 300,000 ms, more preferably from 1,000 ms to 300,000 ms, still more preferably from 1,500 ms to 300,000 ms, yet still more preferably from 1,500 ms to 10,000 ms.

In the concentration process, when a cycle of one voltage application period and one voltage non-application period is defined as a single set, the ratio of the duration of the voltage application period in the single set with respect to the duration of the single set is not particularly restricted. This ratio is hereinafter also referred to as "Duty". The lower limit of the Duty is, for example, preferably 1% or more, more preferably 25% or more, still more preferably 50% or more. The upper limit of the Duty is, for example, preferably less than 100%, more preferably 85% or less, still more preferably 50% or less. The Duty is in a range of, for example, preferably from 1% to less than 100%, more preferably from 1% to 99%, still more preferably from 15% to 85%, particularly preferably from 45% to 55%. The Duty is, for example, preferably 50%.

In the concentration process, the number of times of repeating the voltage application period and the voltage non-application period is not particularly restricted, and it is, for example, from twice to 9,600 times, preferably from 3 times to 5 times.

Regarding the concentration process, the conditions of the voltage application period and the voltage non-application period per one set are exemplified below; however, the invention is not restricted thereto.
Application cycle: from 250 ms to 600,000 ms
Non-application time: from 125 ms to 300,000 ms
Duty: from 1% to less than 100%
Electric current in the voltage application period: from 0.01 mA to 200 mA
Electric current in the voltage non-application period: 0 mA

As described above, the detection process is a process of generating a plasma by applying voltage to the pair of electrodes and detecting light emitted by the analyte due to the plasma.

The detection process may be performed, for example, continuously or discontinuously with the concentration process. In the former case, the detection process is initiated simultaneously with the completion of the concentration process. In the latter case, the detection process is initiated within a prescribed time after the completion of the concentration process. The prescribed time is, for example, from 0.001 to 1,000 seconds, preferably from 1 to 10 seconds, after the completion of the concentration process.

In the detection process, the phrase "generating a plasma" means to substantially generate a plasma, specifically to generate a plasma which causes emission of light that is substantially detectable in the detection of plasma emission. As a specific example thereof, it is regarded that a plasma has been generated when plasma emission is detectable using a plasma emission detector.

Substantial plasma generation can be controlled by, for example, adjusting the voltage. Thus, those of ordinary skill in the art would be able to appropriately set voltage for generating a plasma which causes emission of light that is substantially detectable (hereinafter, also referred to as "plasma voltage"). The plasma voltage is, for example, 10 V or higher, preferably 100 V or higher. The upper limit of the plasma voltage is not particularly restricted and may be, for example, 1000 V or less. The plasma-generating voltage is, for example, relatively higher than the voltage at which the above-described concentration takes place. Therefore, it is preferrable that the plasma voltage is higher than the concentration voltage. The plasma voltage may be, for example, constant throughout the period of the detection process, or may be changed during the detection process.

The duration of applying the plasma voltage (hereinafter, also referred to as "application time" in the detection process) is not particularly restricted and may be set as appropriate in accordance with the plasma voltage. The duration of applying the plasma voltage is, for example, preferably from 0.001 to 0.02 seconds, more preferably from 0.001 to 0.01 seconds. The voltage application to the pair of electrodes may be performed, for example, continuously or discontinuously. The discontinuous voltage application is, for example, pulse application. When the voltage application is performed discontinuously, the duration of applying the plasma voltage may be defined as, for example, the time of performing a single application of the plasma voltage, a total time of applying the plasma voltage, or a total of the time of applying the plasma voltage and the time of not applying the plasma voltage.

In the detection process, the electrode for the plasma generation may be controlled by, for example, allowing the pair of electrodes to have different liquid-contact areas. Specifically, by setting the liquid-contact area of one of the electrodes to be smaller than that of the other electrode, a plasma can be generated on the former electrode. Accordingly, in the present disclosure, it is preferred that each of the pair of electrodes has a different area of contact with the sample; and that, of the pair of electrodes, an electrode having a smaller area of contact with the sample is an electrode for analyzing the analyte by plasma generation. When each of the pair of electrodes has a different liquid-contact area, the difference between the liquid-contact areas of the pair of electrodes is, for example, preferably from 0.001 cm² to 300 cm², more preferably from 1 cm² to 10 cm². In the present disclosure, the term "liquid-contact area" means an area that is in contact with the sample. The method of adjusting the liquid-contact area is not particularly restricted, and examples thereof include a method of immersing different lengths of the electrodes in the sample, and a method of partially coating one or the pair of the electrodes that are in contact with the sample with an insulating material. The insulating material is not particularly restricted, and examples thereof include a resin, silicone, glass, paper, a ceramic, and a rubber. Examples of the resin include a thermoplastic resin such as polyethylene, polypropylene, polystyrene, polyvinyl chloride, polyethylene terephthalate, polymethacrylate, polyamide, a saturated polyester resin, an acrylic resin, polybutylene terephthalate (PBT), polyether ether ketone (PEEK) and polymethylpentene (e.g., TPX^{™}); and a thermosetting resin, such as a urea resin, a melamine resin, a phenol resin, a fluorocarbon resin, an epoxy resin (e.g., glass epoxy) and an unsaturated polyester resin. Examples of the silicone include polydimethylsiloxane.

In the detection process, light emission caused by the generated plasma may be detected, for example, continuously or discontinuously. The detection of the light emission is, for example, detection of the presence or absence of light emission, detection of the intensity of emitted light, detection of a specific wavelength, and/or detection of a spectrum. The detection of a specific wavelength is, for example, detection of a characteristic wavelength generated by the analyte during plasma emission. The method of detecting the light emission is not particularly restricted and, for example, any known optical measuring devices such as a CCD (charge coupled device) or a spectrometer may be employed.

The voltage application to the electrodes may be performed using a voltage application means. Regarding the voltage application means, for example, the above-provided descriptions may be applied. In the detection process, the electric current between the electrodes is, for example, preferably from 0.01 mA to 100,000 mA, more preferably from 50 mA to 2,000 mA.

The analysis method according to the present disclosure may further include a calculation process of calculating the concentration of the analyte in the sample from the detection results obtained in the detection process. Examples of the detection results include the intensity of emitted light. In the calculation process, the concentration of the analyte can be calculated on the basis of, for example, the detection results and the correlation between the detection results and the concentration of the analyte in the sample. The correlation can be determined by, for example, plotting detection results, which are obtained by the analysis method according to the present disclosure for standard samples each having a known concentration of the analyte, and the analyte concentrations of the standard samples. The standard samples are preferably a dilution series of the analyte. By performing the calculation in this manner, highly reliable quantification can be achieved.

In the analysis method according to the present disclosure, the pair of electrodes may be placed inside a container that includes a light-transmitting section. In this case, in the detection process, the emitted light is detected by a light-receiving section which is arranged in such a manner that it can receive light emitted by the analyte via the light-transmitting section. Regarding the container, the light-transmitting section, the light-receiving section and the like, for example, the below-provided descriptions on the analyzer according to the present disclosure may be applied.

### <Plasma Spectroscopic Analyzer>

As described, the plasma spectroscopic analyzer according to the present disclosure is characterized by including: a pair of electrodes; a container; a light-receiving section; and a constant current section, wherein: the container includes a light-transmitting section, the pair of electrodes is placed inside the container, the light-receiving section, which is capable of receiving, via the light-transmitting section, light emitted by an analyte due to voltage application to the pair of electrodes, is placed outside the container, the constant current section is connected to the pair of electrodes and controls an electric current between the pair of electrodes so as to be constant during the voltage application to the pair of electrodes, and the plasma spectroscopic analyzer according to the present disclosure is used for performing the plasma spectroscopic analysis method according to the present disclosure. The analyzer according to the present disclosure is characterized in that it is used for performing the analysis method according to the present disclosure, and other constitutions and conditions are not particularly restricted. According to the analyzer in the present disclosure, the analysis method according to the present disclosure can be carried out easily. Regarding the analyzer according to the present disclosure, for example, the above-provided descriptions on the analysis method according to the present disclosure may be applied.

One example of the analyzer according to the present disclosure will now be described referring to the drawings. In the drawings, for the sake of convenience of description, the structures of the respective components may be shown in a simplified manner as appropriate, or shown schematically, and the scale ratio and the like may be different from the actual state.

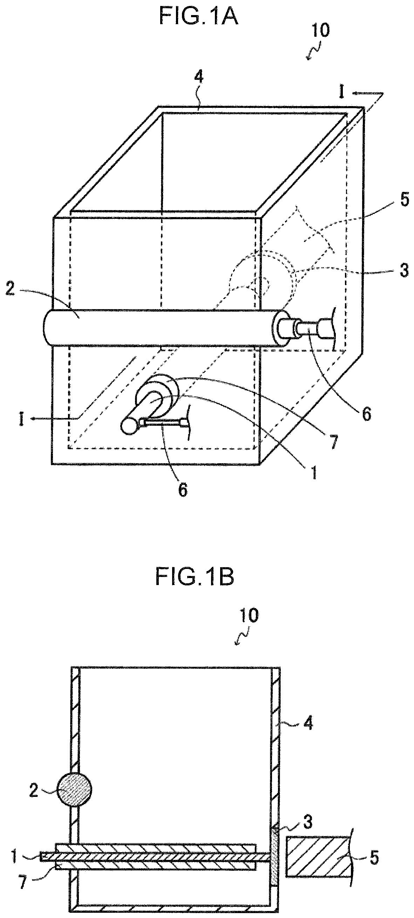

FIG. 1A is a schematic perspective view which illustrates the analyzer according to the present embodiment, and FIG. 1B is a schematic cross-sectional view taken in the direction of I-I as shown in FIG. 1A. As shown in FIGs. 1A and 1B, an analyzer 10 according to the present embodiment includes: a pair of electrodes 1 and 2; a container 4; a light-receiving section 5; and a constant current section 6. The container 4 includes a light-transmitting section 3. The light-receiving section 5, which is arranged such that the light-receiving section 5 can receive, via the light-transmitting section 3, light emitted by an analyte due to voltage application to the pair of electrodes 1 and 2, is placed outside the container 4. The electrode 1 is arranged horizontally to the bottom surface of the container 4, with one end of the electrode 1 being in contact with the light-transmitting section 3. The electrode 2 is arranged horizontally to the bottom surface of the container 4, and arranged on a side surface of the container 4 such that the electrode 2 is partially exposed to the inner side of the container 4. The electrode 1 is coated with an insulating material 7. The constant current section 6 is connected to the electrodes 1 and 2. In the analyzer 10 according to the present embodiment, for example, an analyte-containing sample is introduced into the container 4 in such a manner that the analyte-containing sample comes into contact with the electrodes 1 and 2. In the present embodiment, the analyzer 10 is a vertical placement-type analyzer; however, the analyzer 10 is not restricted to this embodiment and may be, for example, a horizontal placement-type analyzer.

In the present embodiment, the constant current section 6 is not particularly restricted, and any known means for controlling the current value to be constant may be employed, specific examples of which include a constant current source and a galvanostat.

In the present embodiment, the surface of the electrode 1 is partially coated with the insulating material 7; however, the insulating material 7 is an optional component which may be present or absent. Further, in the present embodiment, although the electrodes 1 and 2 are both arranged horizontally to the same plane of the container 4, the arrangements of the electrodes 1 and 2 are not particularly restricted, and the electrodes 1 and 2 may be arranged at any position.

In the present embodiment, the electrode 1 is in contact with the light-transmitting section 3; however, the invention is not restricted to this configuration and, for example, the electrode 1 may be arranged apart from the light-transmitting section 3. The distance between the electrode 1 and a side surface of the container 4 is not particularly restricted, and it is, for example, from 0 cm to 2 cm, preferably from 0 cm to 0.5 cm.

The material of the light-transmitting section 3 is not particularly restricted as long as, for example, it transmits light emitted as a result of voltage application to the pair of electrodes 1 and 2, and the material of the light-transmitting section 3 may be set as appropriate in accordance with the wavelength of the emitted light. Examples of the material of the light-transmitting section 3 include quartz glass, an acrylic resin (e.g., polymethyl methacrylate (PMMA)), borosilicate glass, polycarbonate (PC), a cycloolefin polymer (COP), and a methylpentene polymer (e.g., TPX^{™}). The size of the light-transmitting section 3 is not particularly restricted as long as it is a size which, for example, allows the light emitted as a result of voltage application to the pair of electrodes 1 and 2 to transmit via the light-transmitting section 3.

In the present embodiment, the container 4 has a bottom-closed columnar shape; however, the shape of the container 4 is not restricted thereto and may be any shape such as a bottom-closed cylindrical shape. The material of the container 4 is not particularly restricted, and examples thereof include an acrylic resin (e.g., polymethyl methacrylate (PMMA)), polypropylene (PP), polyethylene (PE), polyvinyl chloride (PVC), polyethylene terephthalate (PET), and polystyrene (PS). The volume of the container 4 may be, for example, from 0.2 cm³ to 3 cm³, or from 0.3 cm³ to 0.5 cm³. When the container 4 has a bottom-closed columnar shape, the width and the depth of the container 4 is, for example, from 0.4 cm to 50 cm, preferably from 1 cm to 5 cm. When the container 4 has a bottom-closed cylindrical shape, the diameter of the container 4 is, for example, from 0.4 cm to 50 cm, preferably from 1 cm to 5 cm. When the container 4 has either a bottom-closed columnar shape or a bottom-closed cylindrical shape, the height of the container 4 is, for example, from 0.3 cm to 50 cm, preferably from 0.5 cm to 5 cm, more preferably from 0.7 cm to 2 cm.

The light-receiving section 5 is not particularly restricted, and examples thereof include known optical measuring instruments such as CCDs and spectrometers. The light-receiving section 5 may be, for example, a transmission means that transmits the emitted light to an optical measuring instrument placed outside the analyzer 10. Examples of the transmission means include a transmission channel such as an optical fiber.

The method for producing the container 4 is particularly restricted and, for example, the container 4 may be produced by injection molding or the like as a molded body, or by forming a recess on a substrate such as a plate. Other examples of the methods of producing the container 4 and the like include, but not particularly limited to, lithography and machining.

### EXAMPLES

Examples of the invention will now be described. It is noted, however, that the invention is not restricted to the following Examples.

### (Example 1)

It was verified that the analysis method according to the present disclosure is capable of analyzing lead contained in urine specimens with only small variations over a wide range of concentration.

### (1) Plasma Spectroscopic Analyzer

The analyzer according to the above-described embodiment was prepared. Specifically, a bottom-closed columnar container made of transparent PMMA (35 mm (height) × 8 mm (width) × 6.5 mm (depth)) was prepared. On a side surface of the container 4, a quartz glass was arranged. The electrodes 1 and 2 were arranged in the container 4 and each connected to the constant current section 6 (galvanostat). The electrodes 1 and 2 were arranged horizontally to the bottom surface of the container 4. The electrode 1 was arranged such that one end thereof was in contact with the quartz glass on the side of the container 4. As the electrode 1, a nichrome electrode rod having a diameter of 0.1 mm and a length of 25 mm was used. A portion having 0.5 mm in length from the end of the electrode 1 were exposed, and the remaining portion was insulated. The electrode 2 was arranged on a side surface of the container 4 in such a manner that the electrode 2 was partially exposed to the inner side of the container 4. As the electrode 2, a carbon electrode rod having a diameter of 4 mm and a length of 15 mm was used. As a light-receiving section, an optical fiber was arranged in such a manner that the optical fiber faces one end of the electrode 1 with the quartz glass therebetween. As the optical fiber, a single-core optical fiber having a diameter of 400 µm was used. The optical fiber was connected to a concave grating spectrometer (self-prepared).

### (2) Plasma Spectroscopic Analysis

After administering EDTA to two subjects by infusion, urine was collected from the subjects and used as urine specimens. To each urine specimen, lithium hydroxide was dissolved such that the concentration becomes 2 mol/L. For each urine specimen, the lead concentration was measured by atomic absorption spectrometry. As a result, the urine specimens were found to have lead concentrations of 2.7 ppb and 12.3 ppb, respectively. As a control (0-ppb specimen), a 2-mol/L aqueous lithium hydroxide solution was used. Next, the three kinds of specimens were each introduced into the container of the analyzer. Then, voltage was applied between the electrodes 1 and 2 under the following concentration conditions such that the electrode 1 served as a cathode and the electrode 2 served as an anode, whereby lead was concentrated in the vicinity of the electrode 1. It is noted here that, in the following concentration conditions, the "application time" represents a total of the time of applying the concentration voltage and the time of not applying the concentration voltage in the concentration process.

### (Concentration Conditions)

Current value during voltage application: 40 mA
Application time: 1,200 seconds
Application cycle: 4 seconds
Duty: 50%

Immediately after the concentration, voltage and an electric current were further applied between the electrodes 1 and 2 under the following plasma generation conditions such that the electrode 1 served as an anode and the electrode 2 served as a cathode, and the emission intensity (number of counts) at a peak near 368.3 nm, which is a wavelength of plasma emission unique to lead, was measured (Example 1, n = 2). It is noted here that, in the following plasma generation conditions, the "application time" represents a total of the time of applying the plasma voltage and the time of not applying the plasma voltage in the detection process.

### (Plasma Generation Conditions)

Applied voltage: 500 V
Application time: 2.5 ms
Application cycle: 50 µs
Duty: 50%

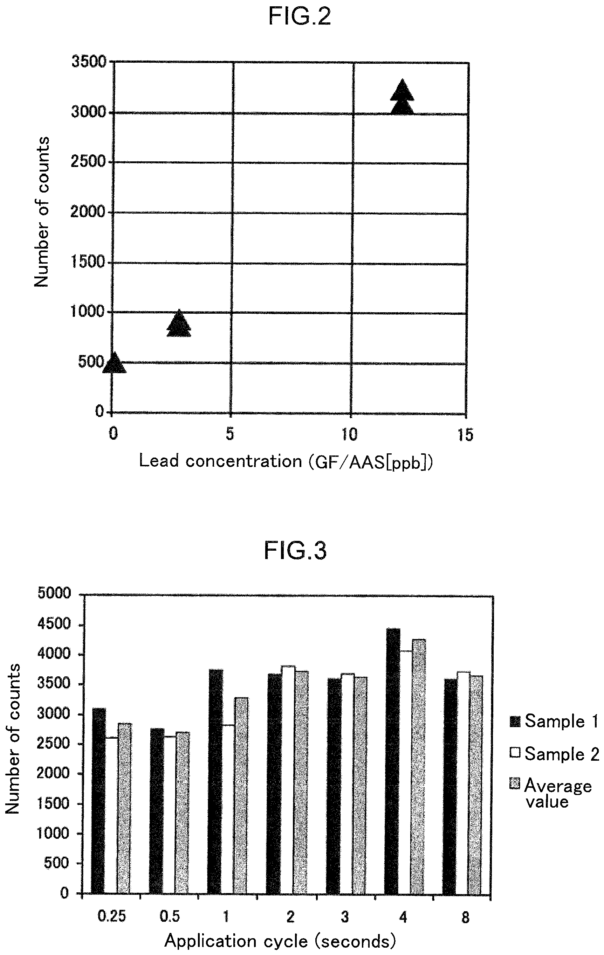

The results of the measurement are shown in FIG. 2. FIG. 2 is a graph showing a correlation between the lead concentration and the number of counts. In FIG. 2, the abscissa axis indicates the lead concentration, and the ordinate axis indicates the number of counts. As shown in FIG. 2, in Example 1, the number of counts increased in a lead concentration-dependent manner, and even a low lead concentration of 2.7 ppb was detected. In addition, the variations in the number of counts between the samples were suppressed. From these results, it was found that, according to the analysis method according to the present disclosure, variations in the number of counts can be suppressed over a wide range of concentration.

### (Example 2)

It was verified that lead contained in urine specimens can be analyzed with only small variations at different application cycles.

The number of counts (n = 2) was measured in the same manner as in the above-described Example 1(2), except that the application cycle of the concentration conditions of Example 1(2) was changed to 0.25, 0.5, 1, 2, 3, 4 or 8 seconds.

The results thereof are shown in FIG. 3. FIG. 3 is a graph showing the number of counts at different cycles. In FIG. 3, the abscissa axis indicates the application cycle, and the ordinate indicates the number of counts. As shown in FIG. 3, an effect was observed even at the application cycle of 0.25 seconds, and the number of counts increased as the application cycle was extended. Further, higher numbers of counts were obtained at application cycles of 2 seconds or longer. From these results, it was found that, according to the analysis method according to the present disclosure, an analyte can be analyzed over a wide range of cycles and, particularly, a strong signal (high number of counts) can be obtained by setting the application cycle to be 2 seconds or longer.

The invention has been described above referring to embodiments and Examples thereof; however, the invention is not restricted to the above-described embodiments and Examples. In the constitutions and details of the invention, various modifications that can be understood by those of ordinary skill in the art may be made within the scope of the invention.

### INDUSTRIAL APPLICABILITY

According to the analysis method according to the present disclosure, for example, occurrences of errors in sample analysis can be suppressed. Further, in the analysis method according to the present disclosure, since occurrences of errors in sample analysis can be suppressed, an analyte can be accurately analyzed regardless of, for example, the type of the sample and the type of the analyte. Moreover, since the analysis method according to the present disclosure can efficiently analyze an analyte locally accumulated at a high concentration without, for example, performing the above-described pretreatment on the sample, the sample can be analyzed by a simple method with high sensitivity. Therefore, the analysis method according to the present disclosure is extremely useful for, for example, the analyses of elements and the like that utilize plasma generation.

### DESCRIPTION OF SYMBOLS

- 1, 2: Electrode
- 3: Light-transmitting section
- 4: Container
- 5: Light-receiving section
- 6: Constant current section
- 7: Insulating material
- 10: Analyzer

## Claims

1. A plasma spectroscopic analysis method, comprising:
a concentration process comprising a voltage application period in which voltage is applied to a pair of electrodes in the presence of a sample, thereby concentrating an analyte, contained in the sample, in the vicinity of at least one of the pair of electrodes; and
a detection process of generating a plasma by applying voltage to the pair of electrodes and detecting light emitted by the analyte due to the plasma,
wherein an electric current between the pair of electrodes is constant while applying voltage for at least a part of the duration of the concentration process.

2. The plasma spectroscopic analysis method according to claim 1, wherein the concentration process further comprises a voltage non-application period in which no voltage is applied to the pair of electrodes.

3. The plasma spectroscopic analysis method according to claim 2, wherein, in the concentration process, when a cycle of one voltage application period and one voltage non-application period is defined as a single set, the duration of the single set is 0.25 seconds or longer.

4. The plasma spectroscopic analysis method according to claim 2 or 3, wherein, in the concentration process, when a cycle of one voltage application period and one voltage non-application period is defined as a single set, the duration of the voltage non-application period in the single set is 0.125 seconds or longer.

5. The plasma spectroscopic analysis method according to any one of claims 2 to 4, wherein, in the concentration process, when a cycle of one voltage application period and one voltage non-application period is defined as a single set, the ratio of the duration of the voltage application period in the single set with respect to the duration of the single set is in a range of from 1% to 99%.

6. The plasma spectroscopic analysis method according to any one of claims 1 to 5, wherein, in the concentration process, the electric current between the pair of electrodes while applying voltage is in a range of from 0.01 mA to 200 mA.

7. The plasma spectroscopic analysis method according to any one of claims 1 to 6, wherein:
each of the pair of electrodes has a different area of contact with the sample, and
of the pair of electrodes, an electrode having a smaller area of contact with the sample is an electrode for analyzing the analyte by plasma generation.

8. The plasma spectroscopic analysis method according to any one of claims 1 to 7, wherein the voltage applied in the detection process is higher than the voltage applied in the concentration process.

9. The plasma spectroscopic analysis method according to any one of claims 1 to 8, wherein the voltage applied in the concentration process is 1 mV or higher.

10. The plasma spectroscopic analysis method according to any one of claims 1 to 9, wherein the voltage applied in the detection process is 10 V or higher.

11. The plasma spectroscopic analysis method according to any one of claims 1 to 10, wherein:
the pair of electrodes is placed in a container,
the container comprises a light-transmitting section, and
a light-receiving section, which is capable of receiving light emitted by the analyte via the light-transmitting section, is placed outside the container.

12. The plasma spectroscopic analysis method according to any one of claims 1 to 11, wherein the analyte is a metal.

13. The plasma spectroscopic analysis method according to claim 12, wherein the metal is at least one selected from the group consisting of aluminum, antimony, arsenic, barium, beryllium, bismuth, cadmium, cesium, gadolinium, lead, mercury, nickel, palladium, platinum, tellurium, thallium, thorium, tin, tungsten, and uranium.

14. The plasma spectroscopic analysis method according to any one of claims 1 to 13, wherein the sample is at least one of a sample derived from a biological source, preferably at least one selected from the group consisting of urine, blood, hair, saliva, sweat, and a nail, or a sample derived from an environmental source, preferably at least one selected from the group consisting of foodstuff, water, soil, the atmosphere, and air.

15. A plasma spectroscopic analyzer for carrying out the plasma spectroscopic analysis method according to any one of claims 1 to 14, the analyzer comprising:
a pair of electrodes;
a container;
a light-receiving section; and
a constant current section, wherein:
the container comprises a light-transmitting section,
the pair of electrodes is placed inside the container,
the light-receiving section, which is capable of receiving, via the light-transmitting section, light emitted by an analyte due to voltage application to the pair of electrodes, is placed outside the container, and
the constant current section is connected to the pair of electrodes and controls an electric current between the pair of electrodes so as to be constant during the voltage application to the pair of electrodes.
